# EUROPEAN PATENT APPLICATION

(11) **EP 2 062 529 A1**
(43) Date of publication of application: **27.05.2009**
(21) Application number: 08169718.7
(22) Date of filing: 21.11.2008
(51) Int. Cl.: A61B 5/0408, A61B 5/0478, A61B 5/0492, A61N 1/04

(54) **Deformable skin electrode and sensor device and corresponding manufacturing method**

(30) Priority: 23.11.2007 GB 0722974
(71) Applicant: Sentrix Technology Limited, Central Hong Kong (CN)
(72) Inventor: LEFTLY, Steven Andrew, Skipton, BD3 6NW (GB); JONES, Dianne Clare, Skipton, BD3 6NW (GB)
(74) Representative: Gibbs, Christopher Stephen

(57) **Abstract**

The present invention relates to a sensor (10) for measuring parameters from a skin surface. The sensor comprises a compressible base (3) having an electrode (12) arranged for contact with the skin surface. The compressible base (3) is resiliently deformable and hence can easily match the contours of the skin surface to which it is to make contact with. This improves the wearer's comfort and the electrical contact between the electrode and the skin surface.

## Description

The present invention relates to a skin sensor device, in particular, though not exclusively, to the design of moulded and formed sensors for use in the field of bio-monitoring.

There currently exist fabric-based sensors that are used for measuring human heart rate and human skin surface parameters by contact with a person's skin. Such existing fabric-based sensors have several problems such as poor skin surface contact and poor comfort. Often there is a problem with electrical contact between the sensor and the skin surface. With existing fabric-based sensors, good electrical contact can only be achieved by using a layer of moisture between the sensor and the skin surface (known as a moisture interface) to create a good electrical pathway.

The present invention has been devised with those problems in mind.

Embodiments of the invention relate to a new sensor design which uses three-dimensional moulded and formed shapes to achieve maximum skin contact and comfort for the user, whilst providing much improved electrical contact with the skin for heart rate monitor sensing and other bio-sensing applications.

Some key (though not essential) points of the invention are:
a three-dimensional moulded electronic sensor system using soft compressible materials,
a manufacturing method for the same, and
the incorporation of the moulded sensor into a bio-monitoring body-worn product.

According to a first aspect of the present invention there is provided a sensor for measuring parameters from a skin surface comprising a compressible base having a flexible electrode arranged for contact with the skin surface. This helps to ensure that good electrical contact is made between the skin surface and the electrode. It also helps to ensure that the sensor is comfortable to wear.

The compressible base may comprise foam. The foam may be EVA, PU or silicone foam. The foam may be thermally compressible. This makes the compressible base easy to manufacture.

It is preferable that the flexible electrode comprises a soft conductive material, a conductive textile, a conductive polymer film or a conductive foam. This makes it easy to manufacture with the base and provides good contact with the skin surface.

The sensor may further comprise a contact point that is in electrical connection with the flexible electrode. This allows signals detected by the sensor to be transmitted to an external device

The compressible base may comprise a raised portion and a flat portion. The flat portion allows the sensor to be attached to a textile with ease. The raised portion may comprise the flexible electrode.

The surface of the sensor that is arranged for contact with the skin surface may be provided with a plurality of protrusions. The electrode may comprise the protrusions. This helps to improve the electrical contact between the electrode and the skin surface.

Preferably the thickness of the sensor is between 5-15mm. This means that it can be worn comfortably and allows it to be integrated into wearable products, for example.

The sensor may be arranged to measure electrical signals of the skin surface. When two sensors are used the potential difference across the skin surface can be detected. This allows a user's heart rate to be monitored.

According to a second aspect of the present invention there is provided a monitoring device comprising a strap having a sensor, according to any to any statement herein, attached thereto. Two sensors may be attached to the strap. The monitoring device may further comprise a wireless transmitter in electrical connection with the or each sensor for wirelessly transmitting signals detected by the or each sensor.

According to a third aspect of the invention there is provided a method of manufacturing a sensor for measuring parameters from a skin surface, comprising forming a compressible base with a flexible electrode arranged for contact with the skin surface. Forming the compressible base may comprise thermally pressing a foam material into a mould. The foam material may comprise a sheet of EVA closed-cell foam.

The invention may comprise any combination of the features and/or limitations referred to herein, except combinations of such features as are mutually exclusive.

Embodiments of the present invention will now be described, by way of example only, with reference to the accompanying drawings, in which:
Figure 1 schematically shows in top view, side view and end view a sensor according to a first embodiment of the present invention;
Figure 2 schematically shows two sensors mounted to a fabric carrier; and
Figure 3 schematically shows in top view only a sensor according to a second embodiment of the present invention.

The sensor system comprises three main components as described below.

### 1. Soft conductive material (flexible electrode)

A conductive material (otherwise known as a flexible electrode) forms either or both surfaces of the sensor. The conductive surface is preferably soft and flexible so that it can adapt to the shape of the skin surface that it makes contact with. This ensures that the sensor is comfortable to wear and also ensures good electrical contact between the electrode and the skin surface.

The conductive material may be constructed from any, or a combination, of the following materials:
a textile fabric containing metallic fibre conductors - woven, knitted, braided or laid in the structure;
a textile fabric containing soft wires - woven, knitted or laid in the structure;
a flexible film or fabric, printed or coated with an electrically conductive material;
a conductive foam material.

The flexible electrode must have a low enough resistance to carry electrical signals from the skin surface to a connector element or contact point.

### 2. Soft moulded form (compressible base)

The conductive material is fixed to a compressible moulded form (otherwise known as a base) which is shaped either before or after the conductive material is fixed to it. The base may be shaped using heat press forming, injection moulding or microinjection moulding depending on the specific design.

The soft moulded form is a three-dimensional shape that is designed to suit specific electrical skin surface contact criteria and also to suit comfort criteria for the user. Shapes such as body shape contours, rounded posts, knobs, ridges or other textures can be moulded into the surface shape to affect this.

The compressible nature of the base means that it can adapt to the skin surface contours against which it is pressed. This makes the sensor comfortable to wear and also ensures good electrical contact between the electrode and the skin surface.

The soft moulded form material may be constructed from any, or a combination, of the following:
heat compressible EVA foam;
moulded PU foam;
moulded or micro-injected rubber.

### 3. Electrical contact points

Contact points are attached to the sensor and are in electrical connection with the conductive material (electrode). These provide a point for signal export to an external electronic device.

The electrical contact points may comprise:
a metallic contact component that is attached to the conductive material at specific locations using a tool (such as a crimp termination component, e.g. Nicomatic Crimpflex (RTM) crimp contacts);
a metallic textile fastener component that is attached to the conductive material at specific locations using a tool. For example, snap fasteners, rivets or other textile fixtures, or conductive Velcro (RTM).

There are a number of advantages of the sensor system over existing technologies:

The sensor is very soft and comfortable for the wearer due to its flexible and compressible nature. This means it is suitable for wearable devices.

The sensor provides good electrical contact between the electrode and the skin surface since the sensor readily conforms to the contours of the surface against which it is held/pressed. This means that sensor can be used on dry skin as opposed to requiring a moisture interface. It also allows the sensor to be used on skin surfaces that are rough or hairy.

The sensor embodied in this invention can be manufactured very economically because of the small number of simple parts.

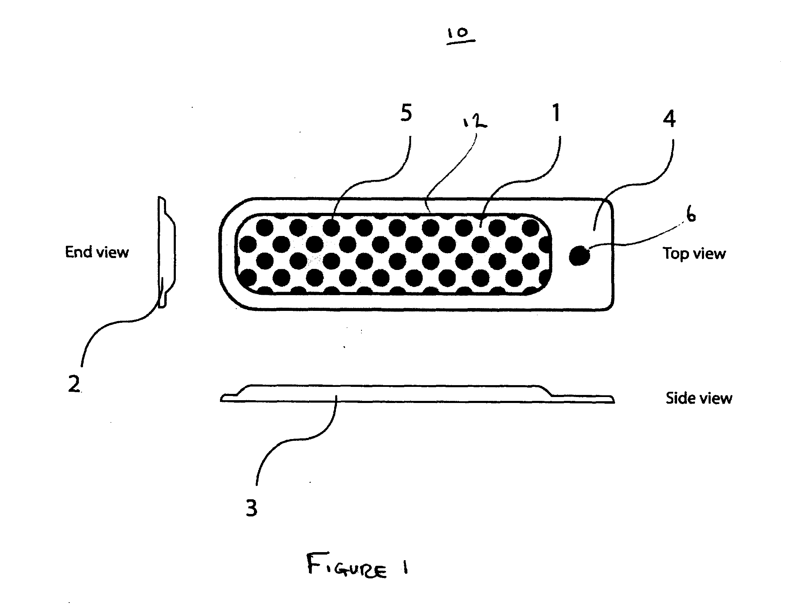

**Figure 1** shows a first embodiment of a heat-pressed EVA moulded sensor 10 for use in a heart rate monitoring product. The sensor comprises a portion of soft conductive material (otherwise known as an electrode 12) having an exposed surface 1. The soft conductive material is moulded into a compressible form (or base) 3. The exposed surface is provided with small further raised areas 5 (or protrusions) and also flat edges 4 (otherwise known as edge regions). These flat edges 4 allow the sensor to be stitched onto a fabric.

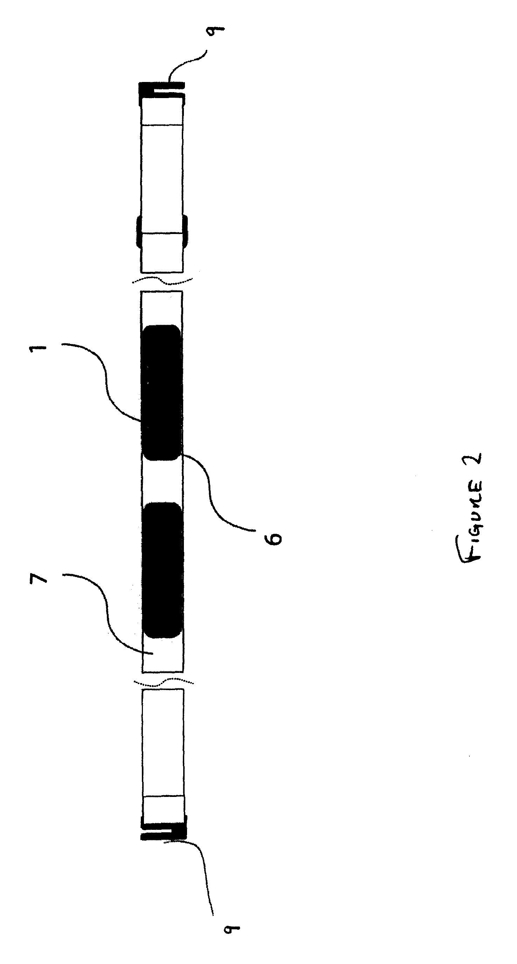

**Figure 2** shows two moulded EVA sensors stitched on to a carrier fabric 7 (otherwise known as a strap). The strap is in the form of a chest strap that can be fastened around the chest of person. This causes the exposed surface 1 of the soft conductive material to be pressed against the chest of the wearer. Electrical contact points 6 are fixed onto the sensors so that signals detected by the sensors can be transmitted to an external device.

In more detail, the sensor 10 comprises a compressible base 3, a flexible electrode 12 comprising a soft conductive material, and a contact point 6. The sensor 10 is a three-dimensional shape having a raised portion and flat portions. The base 3 is a compressible foam that is resiliently deformable. The electrode 12 is in the form of a flexible conductive material and has an exposed surface 1 having a plurality of raised projections 5. In this embodiment the base 3 and electrode 12 are moulded together as one component. A contact point 6 is provided on a flat portion of the sensor and is electrically coupled to the electrode 12.

As shown in Figure 2, two sensors 10 are attached to a fabric strap 7. The flat portions of the sensors allow them to be easily stitched onto the strap 7. The strap 7 has a clip 9 that allows the strap 7 to be attached and fastened around the chest of a user.

In use the strap 7 is fastened around the chest of a user such that the electrodes 12 of both sensors are pressed against the chest of the user. Due to the compressible nature of the base 3 and the flexibility of the electrode 12, the sensor matches the contours of the skin surface it is pressed against. This ensures good electrical contact between the skin surface and the electrode. The sensors 10 are also comfortable to wear due to the compressible base 3 and the flexible electrode 12.

In one embodiment the base 3 and/or electrode 12 is preshaped so as to correspond to the contours of the skin surface against which it is to be positioned.

The electrodes 12 of the sensors 10 are able to detect electrical signals generated by the user's heart. These signals can be fed to an external device (such as a computer, a watch, or any portable device) by wired connection with the contact points 6. Alternatively, the strap 7 can be provided with a wireless transmitter (not shown) that is connected to the contact points 6 to transmit the detected signals to an external device wirelessly.

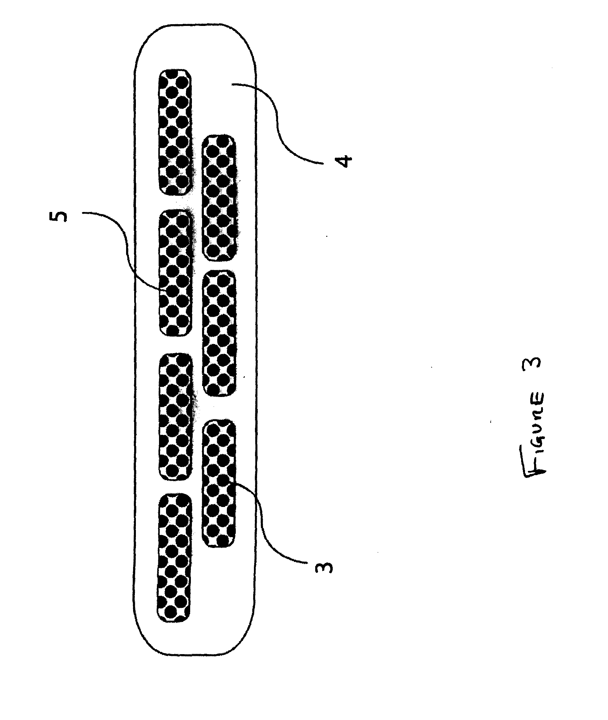

**Figure 3** shows a second embodiment of a moulded EVA form sensor. This sensor is similar to that of the first embodiment except it is provided with multiple raised areas 3 for skin contact and a large flat area 4 that allows the sensor to be stitched onto a fabric.

## Claims

1. A sensor (10) for measuring parameters from a skin surface **characterised by** comprising a compressible base (3) having a flexible electrode (12) arranged for contact with the skin surface.

2. A sensor according to claim 1, wherein the compressible base (3) comprises foam.

3. A sensor according to claim 2, wherein the foam comprises EVA, PU or silicone foam.

4. A sensor according to claim 2 or 3, wherein the foam is thermally compressible.

5. A sensor according to any preceding claim, wherein the flexible electrode (12) comprises a soft conductive material.

6. A sensor according to any preceding claim, wherein the flexible electrode (12) comprises a conductive textile or a conductive polymer film.

7. A compressible sensor according to any of claims 1-4, wherein the compressible base (3) and/or the flexible electrode (12) comprises conductive foam.

8. A sensor according to any preceding claim, wherein the compressible base (3) comprises a raised portion and a flat portion (4).

9. A sensor according to any preceding claim, wherein a surface (1) of the sensor (10) that is arranged for contact with the skin surface is provided with a plurality of protrusions (5).

10. A sensor according to any preceding claim, wherein the thickness of the sensor (10) is between 5-15mm.

11. A sensor according to any preceding claim, wherein the sensor (10) is arranged to measure electrical signals of the skin surface.

12. A monitoring device comprising a strap (7) having a sensor (10) according to any of claims 1-11 attached thereto.

13. A method of manufacturing a sensor (10) for measuring parameters from a skin surface, comprising forming a compressible base (3) with a flexible electrode (12) arranged for contact with the skin surface.

14. A method according to claim 17, wherein forming the compressible base (3) comprises thermally pressing a foam material into a mould.

15. A method according to claim 18, wherein the foam material comprises a sheet of EVA closed-cell foam.
